(19) Europäisches Patentamt
European Patent Office
Office européen des brevets

(11) **EP 4 513 500 A1**

(12) **EUROPEAN PATENT APPLICATION**
published in accordance with Art. 153(4) EPC

(43) Date of publication:
**26.02.2025 Bulletin 2025/09**

(21) Application number: **23915010.5**

(22) Date of filing: **27.12.2023**

(51) International Patent Classification (IPC):
*G16C 20/70* (2019.01)    *G16C 20/40* (2019.01)
*G16C 20/80* (2019.01)    *G06N 3/04* (2023.01)
*G06N 3/08* (2023.01)

(52) Cooperative Patent Classification (CPC):
**G06N 3/04; G06N 3/08; G16C 20/40; G16C 20/70; G16C 20/80**

(86) International application number:
**PCT/KR2023/021741**

(87) International publication number:
**WO 2024/147550 (11.07.2024 Gazette 2024/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.01.2023 KR 20230001253**

(71) Applicant: **LG Chem, Ltd.**
**Yeongdeungpo-gu**
**Seoul 07336 (KR)**

(72) Inventors:
• **MOON, Hee Yeon**
  **Daejeon 34122 (KR)**
• **BAE, Jae Soon**
  **Daejeon 34122 (KR)**
• **LEE, Kyu Hwang**
  **Daejeon 34122 (KR)**
• **SHIN, Hyeon Ah**
  **Daejeon 34122 (KR)**

(74) Representative: **Hoffmann Eitle**
**Patent- und Rechtsanwälte PartmbB**
**Arabellastraße 30**
**81925 München (DE)**

(54) **METHOD AND SYSTEM FOR PREDICTING GLASS TRANSITION TEMPERATURE OF POLYMER**

(57)    The present invention provides a computer-implemented method for estimating a glass transition temperature of a polymer from a chemical structure of the polymer. The present invention provides a method for graphically representing a chemical structure of a polymer, and a method and a system for calculating a glass transition temperature of a polymer from graph data of the polymer by machine-learning a chemical structure of the polymer based on information defining an inter-attachment relationship between respective atoms constituting a repeat unit of the polymer and an attaching node to which repeated repeat units are attached, in which, in addition to the chemical structure of the polymer, molecular weight information is reflected.

**Fig. 5**

POLYMER STRUCTURE INFORMATION,
POLYMER MOLECULAR WEIGHT INFORMATION

↓

DATA INPUT UNIT (100)

POLYMER GRAPH DATA

ARTIFICIAL NEURAL NETWORK MODEL UNIT (200)

POLYMER GLASS TRANSITION TEMPERATURE PROPERTY INFORMATION

GLASS TRANSITION TEMPERATURE CALCULATION UNIT (300)

POLYMER MOLECULAR WEIGHT INFORMATION

POLYMER GLASS TRANSITION TEMPERATURE

**Description**

## TECHNICAL FIELD

The present invention relates to a method and a system for estimating a glass transition temperature of a polymer using an artificial intelligence neural network.

## BACKGROUND ART

In order to input a structure of a compound into a computer device and perform predetermined processing, various methods for representing the structure of the compound as a computer device recognizable one have been suggested. Representative methods include a method using a simplified molecular-input line-entry system (SMILES) for representing the structure of a compound as a string or a method for representing the string converted with the SMILES as a descriptor.

However, it is difficult to represent polymers, which are not single molecules, in the SMILES method, and thus BigSMILES or Hierarchical Descriptor are suggested.

However, in such technologies in the prior art, there are problems in that the larger a molecule is, the more complex the method for representing the molecule, a high molecule is represented only in fragments and thus there is no technology to process the high molecule based on the representation, properties of a polymer in which repeat units are infinitely repeated may not be properly represented, and so on.

Meanwhile, in estimating certain properties of a polymer, in the prior art, as presented in the non-patent documents below, by reflecting structural properties of the polymer, the certain properties of the polymer have been estimated from the reflected properties.

However, among the properties of a polymer, since the glass transition temperature (Tg) is affected not only by the structure of the polymer but also by the molecular weight of the polymerized polymer, there is a problem in that it is not possible to estimate the accurate glass transition temperature from the technologies in the prior art.

The technologies in the prior art are as follows.

Korean Patent Laid-Open Publication No. 2021-0042777 A
Korean Patent Laid-Open Publication No. 2021-0110539 A
Machine-learning predictions of polymer properties with Polymer Genome, Journal of Applied Physics 128, 171104, 2020
Machine learning discovery of high-temperature polymers, Matter, Volume 4, Issue 5, 5 May 2021, pages 1454-1456

## DISCLOSURE OF THE INVENTION

### TECHNICAL PROBLEM

Accordingly, in order to solve the above-described problems, the present invention is intended to provide a method for providing a data structure representing a polymer material as graphic data, and furthermore, estimating a glass transition temperature of a polymer using the data structure representing the graphic data of the polymer, in which a molecular weight element of the polymer is reflected.

### TECHNICAL SOLUTION

In order to solve the above problems, according to the present invention, there is provided a computer-implemented method for graphically describing a chemical structure of a selected polymer and calculating an estimated value of a glass transition temperature of the polymer, the computer-implemented method including a polymer glass transition temperature property information estimation artificial neural network acquisition step of generating a machine-trained graph artificial neural network to estimate glass transition temperature property information about the selected polymer based at least in part on chemical structure data related to the selected polymer, a polymer graph data acquisition step of acquiring polymer graph data describing a chemical structure of the selected polymer as graphic data; a polymer graph input step of providing the acquired polymer graph data as input to the graph neural network, a polymer glass transition temperature property information acquisition step of receiving the polymer glass transition temperature property information of the selected polymer from output of the graph neural network, and a polymer glass transition temperature calculation step of calculating a glass transition temperature of the selected polymer by substituting molecular weight information about the selected polymer into the polymer glass transition temperature property information.

In this case, the polymer graph data describing the chemical structure of the selected polymer as graph data may include at least one of polymer graph node attachment data that is data representing an inter-attachment relationship between nodes corresponding to respective atoms constituting a repeat unit for composing the polymer and attaching nodes that are attached to an attach point that is a point where the repeat units are repeatedly attached among the nodes, polymer node attribution information that is information representing attribute information about the nodes corresponding to the respective atoms constituting the repeat unit for composing the polymer and attributes of attaching nodes that are attached to the attach point that is a point where the repeat units are repeatedly attached among the nodes, and polymer edge attribute information that is information representing attributes of an edge for attaching the nodes corresponding to the respective atoms constituting the repeat unit for composing the polymer to each other and an attach edge for attaching at least one of the nodes to the attaching node.

According to the present invention, there is also provided a glass transition temperature calculation system for polymers, including a data input unit that receives polymer graph data describing a chemical structure of a selected polymer as graphic data and molecular weight data of the polymer, an artificial neural network model unit that estimates and calculates glass transition temperature property information about the polymer from the polymer graph data, and a glass transition temperature calculation unit that calculates a glass transition temperature of the polymer from the glass transition temperature property information about the polymer and the molecular weight data of the polymer, and in this case, the glass transition temperature property information about the polymer is $T_{g,\ inf}$ value and K in the equation below, the molecular weight data of the polymer is Mn in the equation below, and the glass transition temperature calculation unit calculates a glass transition temperature Tg from the equation below.

$$T_g = T_{g,inf} - \frac{K}{M_n}$$

(Tg is a glass transition temperature of a polymer with a molecular weight Mn, $T_{g,inf}$ is a glass transition temperature when the molecular weight is infinite, K is a variable related to a free volume of the polymer, and Mn is a number average molecular weight of the polymer).

**ADVANTAGEOUS EFFECTS**

According to the present invention, by providing a data structure that can represent a polymer in computer-usable graphical data, it is possible to implement a method and a system for estimating property information about a polymer material by machine-learning a relationship between a molecular structure and property information about the polymer material through a graph neural network (GNN) and a message passing neural network (MPNN). Furthermore, by reflecting molecular weight factors in estimating a glass transition temperature of a polymer, it is possible to provide a method and a system for estimating a glass transition temperature of a polymer with improved accuracy as compared with the prior art.

**BRIEF DESCRIPTION OF THE DRAWINGS**

The following drawings attached to the specification illustrate preferred embodiments of the present invention by example, and serve to enable technical aspects of the present invention to be further understood together with detailed description of the invention given above, and therefore the present invention should not be interpreted only with matters in such drawings.

FIG. 1 is a diagram illustrating graphical representations of monomers and polymers according to the prior art.
FIG. 2 is a diagram illustrating graphical representations of a polymer according to the present invention.
FIG. 3 is an exemplary diagram for describing the graphical representation of the polymer according to the present invention.
FIG. 4 is a diagram illustrating a procedure for implementing an artificial neural network for calculating a glass transition temperature of a polymer using a graphical representation of a polymer according to the present invention.

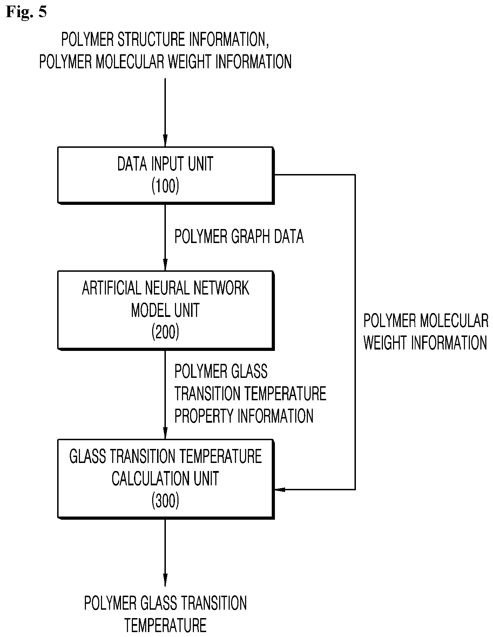

FIG. 5 is a block diagram of a system for estimating a glass transition temperature of a polymer according to the present invention.

**MODE FOR CARRYING OUT THE INVENTION**

1. Graph representation method for polymer according to the present invention

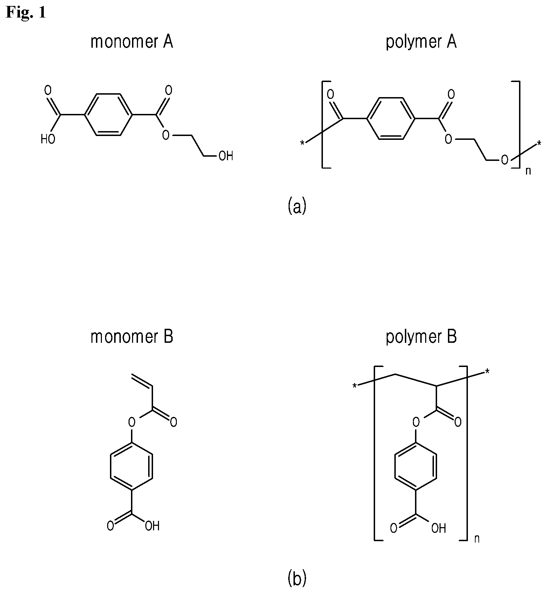

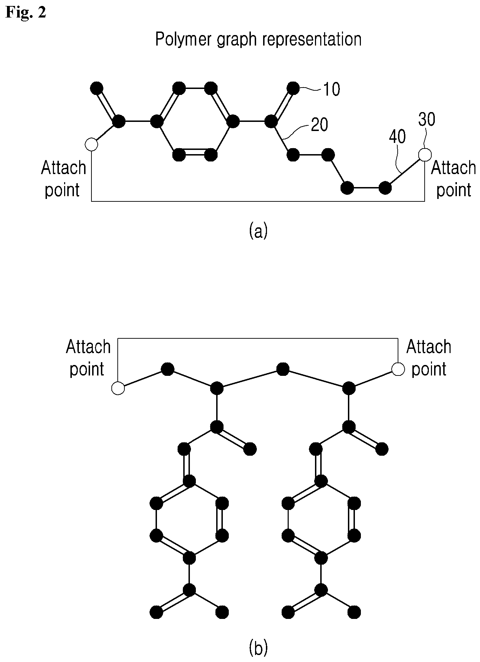

First, a method of graphically representing polymers according to the present invention will be described with reference to FIGS. 1 and 2.

(a) and (b) in FIG. 1 are diagrams illustrating structures of different polymers and the monomers composing each polymer. However, in trying to process molecular structure data using an artificial neural network, especially in representing a molecular structure as a graph for application to a GNN or the like, there is no method of effectively representing a chemical structure of a polymer as a graph.

Monomers that compose polymers illustrated in (a) and (b) in FIG. 1 are different from each other, but since they are the same except for some of the monomer terminals, graphs of the monomers are represented very similarly, and thus it is inaccurate to represent graphs of the polymers simply as graphs of the monomers and to represent the graphs of the polymers as repetitions of the graphs of the monomers.

Therefore, the present invention suggests a new method of representing a polymer graph as illustrated in FIG. 2. Hereinafter, a polymer graph data method according to the present invention will be described with reference to FIG. 2.

First, in a polymer graph representation method of the present invention, monomers for composing a polymer and a repeat unit that is repeated to compose the polymer are derived. FIG. 1 illustrates an example of polymer representation in the prior art and a representation of the monomers for composing the polymer, and FIG. 2 is a diagram illustrating the repeat units for composing the polymer of FIG. 1. In the repeat unit of FIG. 2, for the polymer representation method of the present invention, each atom is expressed as a node 10, and coupling between the atoms is expressed as an edge 20. Next, when the repeat unit is repeated to compose the polymer, which is one of the features of the present invention, a point where the repeat units are repeatedly attached is expressed as an attach point 30, and a node to which the attach point is attached is expressed an "attaching node." In addition, the attach point 30 is a virtual representation of the attaching nodes of neighboring repeat units attached by repetition, and the attachment for attaching the attach point to the attaching node is expressed as an attach edge 40.

According to the polymer graph representation method of the present invention as described above, when an artificial neural network model is trained based on chemical structures of polymers, in particular, when learning is performed by applying an artificial neural network model such as a message passing neural network (MPNN) and a graph neural network (GNN) based on message passing, even by adding a minimal amount of information in addition to monomer structure information, learning to be performed by transferring information about an opposite node where a monomer is attached is possible and learning to be performed by reflecting the unit repetition characteristics of the polymer is possible.

2. Computer-usable datafication of graph representation of polymer according to the present invention

The present invention converts the chemical structure of a polymer into a data structure capable of being used in computer processing, that is, "graph data" according to the graph representation method that graphically represents the above-mentioned molecular or chemical structure of the polymer. In the present invention, this is referred to as "polymer graph data".

According to the present invention, polymer graph data that describes a chemical structure of a given or selected polymer in graphic data may include at least one of node data that is data of nodes 10 representing two or more nodes representing respective atoms constituting a repeat unit of a monomer for composing the polymer and data related to the nodes 10, edge data that is data related to one or more edges 20 representing coupling between respective nodes, attaching node data that is data related to at least one attaching node that is a point where repeat units for composing the polymer are repeatedly attached among the nodes, and attach edge data that is data related to the attach edge 40 attaching the attach point 30 to the attaching node.

A difference between the monomer graph data and the polymer graph data of the present invention is that the polymer graph data of the present invention represents the node 10 attached to the attach point 30 and its edge information as the attaching node and the attach edge in the graph data representation of the repeat unit including the monomer graph data.

2.1. Adjacency matrix and feature matrix

When converting the graph structure of the monomer into data capable of being used on a computer, there may be various data representation methods, but typically, edge data and node data may be represented as an adjacency matrix and a feature matrix, respectively. The edge data is data related to coupling between atoms, and the node data is data related to each atom.

According to a polymer representation method according to the present invention, in addition to a monomer graph representation method in the prior art, the polymer graph is able to be converted into computer-recognizable data by additionally adding data for the nodes and the attach edge 40 attached to the attach point 30.

(1) Adjacency matrix expression of monomer graph

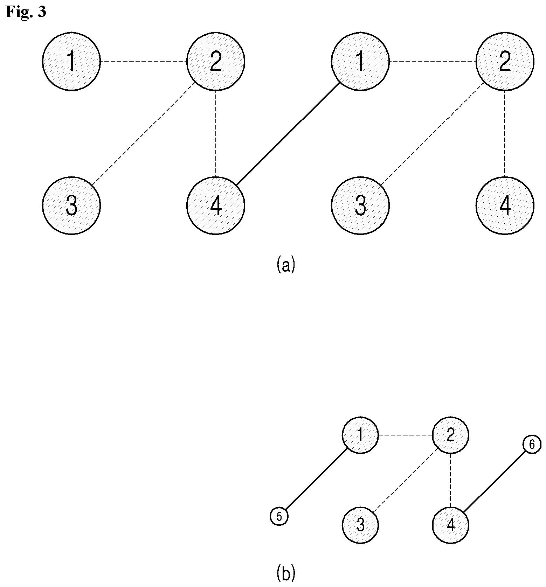

For the sake of simplicity of description, when a simple structure, as illustrated in (a) of FIG. 3, is described by way of example, an adjacency matrix A of a repeat unit of a monomer consisting of nodes 1, 2, 3, and 4 may be represented as follows.

For the sake of simplicity of description, when a simple structure, as illustrated in FIG. 3, is described by way of example, an adjacency matrix A of a repeat unit of a monomer consisting of nodes 1, 2, 3, and 4 may be represented as follows.

$$Monomer\ adjacency\ matrix\ A = \begin{bmatrix} 0 & 1 & 0 & 0 \\ 1 & 0 & 1 & 1 \\ 0 & 1 & 0 & 0 \\ 0 & 1 & 0 & 0 \end{bmatrix}$$

The adjacency matrix A has as many rows and columns as the number of nodes, and each element $A_{i,j}$ is data indicating whether an i (i=1, ..., 4)-th node and a j (j=1, ..., 4)-th node are attached to each other. A value of each matrix element represents data on attachment to other nodes. For example, since a node 1 is only attached to a node 2 and not to nodes 1, 3, and 4, $A_{1,j}$ (j=1, ..., 4) of the adjacency matrix = [0 1 0 0] is represented.

(2) Adjacency matrix expression of polymer graph according to the present invention

According to the polymer graph data representation method of the present invention, a polymer adjacency matrix PA, which is an adjacency matrix representation of the polymer graph according to the example of FIG. 3, may be expressed as follows.

$$Polymer\ adjacency\ matrix\ PA = \begin{bmatrix} 0 & 1 & 0 & \mathbf{1} \\ 1 & 0 & 1 & 1 \\ 0 & 1 & 0 & 0 \\ \mathbf{1} & 1 & 0 & 0 \end{bmatrix}$$

As illustrated in (b) of FIG. 3, the polymer adjacency matrix PA has two attach points (5, 6) added, and these points refer to nodes 4 and 1 of an adjacent monomer, respectively, and thus the nodes 1 and 4 are represented as being attached to nodes 4 and 1, respectively. That is, since there is no node attached to nodes 1 and 4 in the monomer, matrix values indicating attachment relationships of the node 1 and the node 4 are $A_{1,4}= 0$, $A_{4,1}=0$, but in the polymer representation method of the present invention, since the nodes 1 and 4 are attached to nodes 4 and 1 of adjacent monomers, respectively, the polymer adjacency matrix PA is expressed as $PA_{1,4}=1$, $PA_{4,1}=1$, and contains repetitive attach data of the polymer. That is, since the nodes 1 and 4, which have not been attached in the monomer, are attached to each other in the polymer, the nodes 1 and 4 are represented as being attached to each other in the polymer adjacency matrix that displays the attach edge data.

However, in this case, since the nodes 1 and 4 are not attached to each other within the monomer, in order to indicate that the attachment is not attachment within the monomer, but attachment to the node of the adjacent repeat unit, a distinction may be made by expressing the node attachment relationship as a negative number to express the polymer adjacency matrix as follows, giving a feature of "attaching node attached to the attach point" to the feature of the corresponding node in a node feature matrix to be described below, or giving an adjacent repeat unit attachment attribute to an edge feature matrix.

$$Polymer\ adjacency\ matrix\ PA' = \begin{bmatrix} 0 & 1 & 0 & -1 \\ 1 & 0 & 1 & 1 \\ 0 & 1 & 0 & 0 \\ -\mathbf{1} & 1 & 0 & 0 \end{bmatrix}$$

In other words, the graph data that represents the chemical structure of the polymer as a graph and describes it as graphic data according to the present invention includes, in addition to the graph data of the repeat unit of the monomer, attaching node data representing an attaching node that is a node to which the monomers are repeatedly attached and data of the attach edge 40 that is attached to the attaching node.

The expression method of the polymer adjacency matrix PA indicates that the nodes 1 and 4 of the repeat unit of the monomer consisting of nodes 1, 2, 3, and 4 are attached to the nodes 4 and 1 of the neighboring repeat unit, respectively, and this attachment is repeated. However, at this time, the nodes 1 and 4 in FIG. 3 are not attached to each other within the monomer, but are attached to the nodes of the adjacent repeat unit, so that these nodes may be distinguished by designating the nodes as "attaching nodes" and giving the attaching node attribute to the node feature matrix or giving the adjacent repeat unit attachment attribute to the edge feature matrix.

This may be used as an embedding procedure to convert the chemical structure of a polymer into data capable of being processed by a computer, which is achieved by a polymer edge variable setting step of setting an inter-attachment relationship between nodes representing respective atoms constituting the monomer repeat unit of the selected polymer and the attaching nodes that are points where the monomers are repeatedly attached as a polymer attachment edge variable, and a polymer graph edge data assignment step of assigning an attribute value of the inter-attachment relationship between the nodes and the attaching nodes to the set polymer edge variable. As seen in the polymer adjacency matrix PA above, the attribute value of the inter-attachment relationship between the nodes and the attaching nodes may be determined as "1" and "0", indicating attachment/non-attachment, the matrix expression of which is the polymer adjacency matrix. The polymer adjacency matrix of the present invention has the same component values as a typical adjacency matrix, but as described above, it differs from the typical adjacency matrix by adding attaching nodes to a typical single molecule graph node.

(3) Feature matrix of monomer graph

In addition to the adjacency matrix showing the attachment relationship between nodes, the graph representation of a compound may have a feature matrix representing predetermined attribute information about each node and predetermined attribute information about each edge as graph data. In an exemplary monomer graph of FIG. 3, a monomer node feature matrix containing attribute value information (e.g., three pieces of attribute information) about each atom can be expressed as follows.

$$\text{Monomer node feature matrix } PA' = \begin{bmatrix} 0 & 1 & 0 \\ 1 & 0 & 1 \\ 0 & 1 & 0 \\ 0 & 1 & 0 \end{bmatrix}$$

A node feature matrix NF may have as many rows as the number of nodes and as many columns as the number of types of feature values to be expressed for each node, and thus may express a certain attribute to be expressed for each atom. Each component has a j-th feature value of an i-th node. For the purpose of description, the monomer feature matrix NF is described as an example in which each node has three arbitrary attribute values.

Information of the monomer graph may be represented as an edge feature matrix EF, where each row may represent each edge of a monomer and column data of each row may be represented to express a predetermined attribute value of each edge. Edge attribute values may include the type of chemical bond (single bond, double bond, or the like), a ring status, a conjugation status, or the like. For example, in the case of the monomer in FIG. 3, since there are three attach edges, the edge feature matrix has three rows, and edge attribute values intended to contain information are assigned to respective columns. An example of the edge feature matrix with three random edge attribute values is as follows.

$$\text{Monomer edge feature matrix } EF = \begin{bmatrix} 1 & 0 & 0 \\ 0 & 1 & 1 \\ 0 & 0 & 0 \end{bmatrix}$$

(4) Feature matrix of polymer graph according to the present invention

Polymer graph data according to the present invention may have separate attribute values added to the attribute values of the monomer node feature matrix described above. For example, a polymer node feature matrix PNF, which is a feature matrix of the exemplary polymer graph in FIG. 3, may also have three columns plus attribute data, as in the example illustrated below, and express the added column as attribute values indicating whether respective nodes are an attaching node. For example, the polymer node feature matrix PNF below may have attaching node attribute information in a fourth column, as illustrated below, in addition to the monomer node feature matrix NF containing three pieces of attribute information about the previous four nodes. In the example of FIG. 3, since the nodes 1 and 4 are attaching nodes attached to adjacent repeat units, the nodes 1 and 4 are represented as having a value of "1", and other nodes are represented as having a value of "0" in the fourth column.

$$Polymer\ node\ feature\ matrix\ PNF = \begin{bmatrix} 0 & 1 & 0 & \mathbf{1} \\ 1 & 0 & 1 & 0 \\ 0 & 1 & 0 & 0 \\ 0 & 1 & 0 & \mathbf{1} \end{bmatrix}$$

This may be achieved by a polymer node variable setting step of setting each of atoms of a monomer for composing a selected polymer and an attaching node that is a point to which the monomers are repeatedly attached as polymer node variables and a polymer graph node data assignment step of assigning an attaching node to be attached to the set polymer node variable and assigning a node attribute value to each of the nodes. The node attribute values assigned to nodes may include atomic numbers, hybridizations (SP3, SP2, or the like), the number of hydrogens, the number of electrons, ring status, or the like, and in particular, as one embodiment of the present invention, may have an attaching node attribute value, which is an attribute value of whether a node is attached to an adjacent repeat unit.

As another embodiment of the present invention, attaching node data may be represented by an edge feature matrix. For example, like the polymer node feature matrix described above, instead of expressing with a node attribute value whether a node is an attaching node, or together with the node attribute value, an attribute value expressing whether a node is an attaching node is expressed in the edge feature matrix. In the example below representing (b) in FIG. 3, it can be seen that data in fourth and fifth rows are added to contain information on the edges attached to the attaching nodes 1 and 4, that is, edges 5_1 and 4_6 in (b) in FIG. 3 and an attribute information column expressing whether a node is an attaching node is added in the fourth column.

$$Polymer\ edge\ feature\ matrix\ PEF = \begin{bmatrix} 1 & 0 & 0 & \mathbf{0} \\ 0 & 1 & 1 & \mathbf{0} \\ 0 & 0 & 0 & \mathbf{0} \\ 0 & 0 & 0 & \mathbf{1} \\ 0 & 0 & 0 & \mathbf{1} \end{bmatrix}$$

That is, according to the present invention, the method of representing the structure of a polymer as a graph and converting it into computer-usable data may be selected from one or more of the methods below.

① In the adjacency matrix expressing the attachment relationship between respective nodes of a repeat unit formed by at least one monomer, a component of an "attaching node" attached to an adjacent repeat unit is assigned as an "attachment" attribute.

② An "attachment status" attribute field is given to a node attribute matrix expressing attributes of respective nodes of the repeat unit, and "attachment" is assigned to the "attachment status" attribute field of the "attaching node".

③ Edges attached to the attaching node are added as attach edges to the edge attribute matrix of the repeat unit, "attachment status" attribute fields are given to the edge attributes, and "attachment" is assigned to the corresponding fields of the attach edges.

2.2. Method of converting chemical structure of polymer into data capable of being processed by computer

In the present invention, in training the artificial neural network model to be described below and estimating a property of a polymer material using the trained artificial neural network, the chemical structure of the polymer material has to be converted into data so that the polymer may be processed by a computer, and the conversion into data may be performed as follows according to a method of the present invention.

(1) Repeat unit information acquisition step

This is a step of acquiring information on the repeat unit, which is a unit for a polymer composed by repeating a monomer. In order to convert attaching node data in the repeat unit into data, which is one of the features of the present invention, the information on the repeat unit for composing the polymer is acquired. This is a step to confirm whether the repeat unit repeated to compose the polymer is repeated as a single monomer or as a combination of single monomers, and to describe the repeat unit in a graphical representation. The repeat unit may be a single monomer as shown in (a) in FIG. 2 or may be a case where monomers are attached to each other as shown in (b) in FIG. 2.

(2) Polymer graph node attachment information assignment step

In the present invention, a method for processing polymer graph data includes a step of setting, as a variable, an inter-attachment relationship between nodes representing respective atoms constituting a repeat unit of a polymer selected as an analysis target and attaching nodes attached to an attach point that is a point where the repeat units are repeatedly attached among the nodes.

An attribute value of the inter-attachment relationship between the nodes and attaching nodes is assigned to the polymer node attachment variable set in this way. To assign the attribute value of each attachment relationship to the polymer node attachment variable, the polymer adjacency matrix mentioned above may be utilized.

(3) Polymer graph node attribute information assignment step

The method for processing polymer graph data of the present invention may also include a polymer node attribute variable setting step of setting, as polymer node attribute variables, attribute information about nodes corresponding to respective atoms constituting the repeat unit for composing the selected polymer and attribute information about the attaching nodes attached to the attach point that is a point where the repeat units are repeatedly attached among the nodes.

The method additionally includes a polymer graph node attribute information allocation step of assigning attribute values of the nodes and attaching nodes to the polymer node attribute variables set in this way. The node attribute information has an attribute value indicating whether the node is an attaching node or not, and as node attribute information for the attaching node, unlike nodes that are not the attaching node, an attaching attribute value indicating that the node is attached to the attaching node of neighboring repeat unit within the polymer is given.

(4) Polymer graph edge attribute information assignment step

The method for processing polymer graph data of the present invention may also include a polymer edge attribute variable setting step of setting, as polymer edge attribute variables, information about edges inter-attaching nodes corresponding to respective atoms constituting the repeat unit for composing the selected polymer and an attach edge attaching at least one among the nodes to the attaching node. The attach edge is an edge attaching the attaching node to another node.

The present invention also includes a polymer graph edge attribute information assignment step of assigning attribute values of the edges and the attach edges to the set polymer edge attribute variables, and the attach edge is assigned an attach edge attribute value, which means that the edge attaches the attaching node to another node, as its edge attribute variable.

3. Method for estimating glass transition temperature of polymer using graph representation of polymer according to the present invention

Hereinafter, a method for processing polymer data in an artificial intelligence neural network according to the polymer graph representation method of the present invention described above will be described.

The present invention provides a method for constructing an artificial neural network model that estimates a glass transition temperature of a polymer by applying the above-described polymer graph representation method and estimating the glass transition temperature of the polymer through machine learning.

3.1. Polymer glass transition temperature estimation model generation method

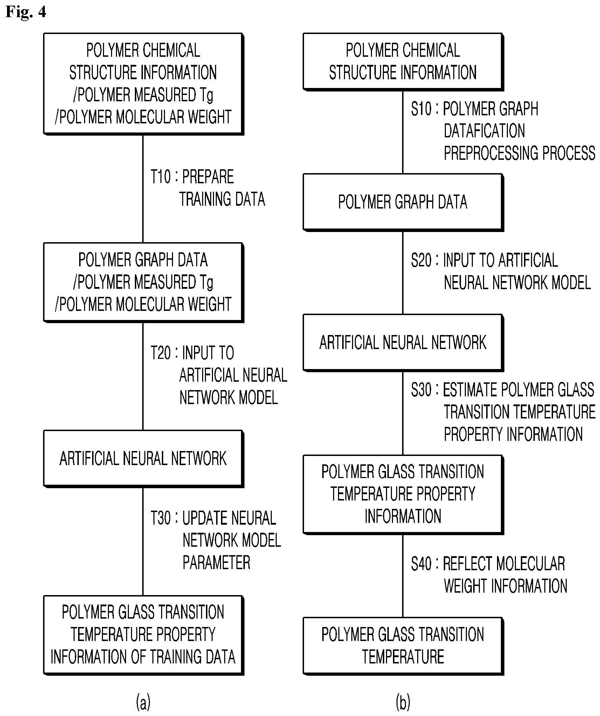

(a) in FIG. 4 illustrates a polymer glass transition temperature estimation model generation method according to the present invention. Based on this, a method for generating a computer-implemented polymer glass transition temperature estimation model for estimating a glass transition temperature of a selected polymer from a chemical structure of the corresponding polymer according to the graph data representation method of the present invention will be described.

The polymer glass transition temperature estimation model of the present invention is configured to estimate the glass transition temperature by applying the relationship between the glass transition temperature and molecular weight of a polymer defined by the Flory-Fox Equation of [Equation 1]. The glass transition temperature Tg of a polymer is a physical property value that is affected by both the molecular structure and the molecular weight, but the technology in the prior art fails to reflect the effects of both the molecular structure and the molecular weight in estimating the glass transition temperature. The present invention has implemented an estimation model with improved accuracy by reflecting both the molecular structure and the molecular weight in estimating the glass transition temperature using the equation below. In addition, it has become possible to estimate Tg according to changes in molecular weight for polymers of the same

molecular structure.

[Equation 1]

$$T_g = T_{g,inf} - \frac{K}{M_n}$$

$T_{g,inf}$ is a glass transition temperature when the molecular weight is theoretically infinite, K is an experimental parameter related to a free volume in the polymer, and Mn is a number average molecular weight of the polymer.

Accordingly, the estimation model of the present invention includes an artificial neural network that estimates $T_{g,inf}$ and K in Equation 1 from the molecular structure of a selected polymer, and is configured to calculate the glass transition temperature Tg of the selected polymer by applying the $T_{g,inf}$ and K values output by the artificial neural network and the molecular weight of the selected polymer to Equation 1. In this estimation model, the following procedure is performed to generate the artificial neural network model that estimates $T_{g,inf}$ and K values, which are glass transition temperature property information about the polymer.

(1) Training data preparation step (T10)

In order to generate the polymer glass transition temperature estimation model described above, training data for training the artificial neural network is prepared. The training data of the present invention contains a data set of {polymer graph data containing structure information about a sample polymer, a measured Tg value of the sample polymer, and a molecular weight Mn of the sample polymer}.

In addition, the training data of the present invention includes a predetermined number of data sets of {polymer graph data containing structure information about the sample polymer, the measured Tg value of the sample polymer, and the molecular weight Mn of the sample polymer} or more as a data set of a polymer with a similar structure but different molecular weight and a data set of a polymer with a different structure but the same molecular weight. By building the training data set in this way, the artificial neural network of the present invention, which will be described below, may be trained so that the estimated Tg value (Tg estimated value) reflects both the structural features and molecular weight features of the polymer.

(2) Artificial neural network model construction and learning data input step (T20)

Next, the artificial neural network model to be trained using the training data is constructed. The artificial neural network in the present invention uses known artificial neural networks, and in particular, artificial neural networks such as a message passing neural network (MPNN) and a graph neural network (GNN) based on message passing may be applied.

For example, when the GNN is applied as the artificial neural network, describing a case of supervised learning, polymer training data containing data sets of {polymer graph data containing structure information about the sample polymer, the measured Tg value of the sample polymer, and the molecular weight Mn of the sample polymer} built above is input into the GNN.

In this case, polymer graph data is input as an input value of the artificial neural network, and the known measured Tg value and molecular weight Mn of the corresponding polymer are input as true values (labeled data) of the output value of the neural network. In this case, the input polymer graph data may include at least one of the polymer graph node attachment information, the polymer graph node attribute information, and the polymer graph edge attribute information, which have been described above, and may be input by being embedded in the polymer adjacency matrix and polymer node/edge feature matrix.

(3) Artificial neural network training step (T30)

This is a process of training the artificial neural network by updating neural network parameters of the artificial neural network based on the input polymer graph data, the molecular weight information, and glass transition temperature property information about the polymer. The learning process of the artificial neural network follows the known artificial neural network learning process, and as one example, the Tg estimated value is calculated by receiving polymer graph data of the training data and reflecting measured molecular weight information in the training data sets to an estimated value of the glass transition temperature property information about the polymer that is calculated from the artificial neural network according to the [Equation 1], and the neural network parameters of the artificial neural network are updated so that an error function defined as a difference between the Tg estimated value and the measured Tg value of the corresponding polymer in the training data is minimized. Through updating of the neural network parameters, the artificial neural network that calculates glass transition temperature property information from the structure of the polymer is completed.

As such, in the present invention, accurate Tg estimation in the polymer is possible because the artificial neural network is trained to minimize the error between the measured value and the Tg estimated value reflecting molecular weight information.

Meanwhile, in the present invention, the term "neural network parameter" of the artificial neural network contains weights and biases that are adjusted while the artificial neural network model is trained from training data, and such a parameter is adjusted so that the neural network model estimates a more accurate output from input data, and is used in the general sense of the term in the field to indicate that it is optimized to estimate optimal output during a machine learning process.

3.2. Polymer glass transition temperature estimation method

A procedure will be described for estimating a glass transition temperature of a selected polymer by inputting graph data of the selected polymer into the artificial neural network generated according to the polymer glass transition temperature model generation method of the present invention and reflecting the number average molecular weight Mn of the selected polymer.

(1) Polymer graph datafication preprocessing process (S10)

As illustrated in (b) FIG. 4, a preprocessing process for acquiring polymer graph data from the chemical structure of the selected polymer whose glass transition temperature is to be estimated is performed. As described above, the polymer graph data includes data on attaching nodes and the attach edges 40 between repeating monomers, in addition to node and edge data of the monomer for composing the polymer. That is, the polymer graph data is preprocessed to include at least one of the above-described polymer graph node attachment information, polymer graph node attribute information, and polymer graph edge attribute information.

(2) Polymer graph data input step (S20)

This is the step of inputting the polymer graph data based on the chemical structure of the selected polymer into the trained learned neural network model. In this case, the input polymer graph data may include at least one of the polymer graph node attachment information, polymer graph node attribute information, and polymer graph edge attribute information, and may be input by being embedded in the polymer adjacency matrix and polymer node/edge feature matrix described above.

(3) Polymer glass transition temperature property information estimation step (S30)

This is a step in which the trained artificial neural network model calculates and outputs the glass transition temperature property information $T_{g, \, inf}$ and K values of the selected polymer from the input polymer graph data of the selected polymer.

(4) Molecular weight information reflection step (S40)

This is a step of calculating the glass transition temperature Tg using the above [Equation 1] from the glass transition temperature property information $T_{g, \, inf}$ and K values calculated by the artificial neural network model. The molecular weight of the polymer is acquired and reflected by a known method.

4. A system for calculating a glass transition temperature of a polymer using a graph representation of the polymer according to the present invention

(1) Data input unit 100

A data input unit 100 is configured to receive polymer graph data that describes a chemical structure of a selected polymer as graphic data and molecular weight data of the polymer, and further includes a data preprocessing unit (not illustrated) for converting the chemical structure of the polymer into the polymer graph data and inputting the converted data into the data input unit. Molecular weight data (molecular weight information) may be acquired by known methods.

In inputting the polymer graph data into an artificial neural network model unit, the data preprocessing unit may generate and input a polymer adjacency matrix expressing the inter-attachment relationship between two or more nodes representing respective atoms constituting the single molecule and attachment relationship information between the attaching node and the nodes and a polymer edge attribute matrix including attribute information about one or more edges representing coupling between the respective nodes and an attach edge representing attachment between the nodes and the attaching node.

In this case, as described above, the polymer graph data may include at least one of polymer graph node attachment data that is data representing an inter-attachment relationship between nodes corresponding to respective atoms constituting a repeat unit for composing the polymer and attaching nodes that are attached to an attach point that is a point where the repeat units are repeatedly attached among the nodes, polymer node attribution information that is data representing attribute information about the nodes corresponding to the respective atoms constituting the repeat unit for composing the polymer and attributes of attaching nodes that are attached to the attach point that is a point where the repeat units are repeatedly attached among the nodes, and polymer edge attribute information that is information representing attributes of an edge for attaching the nodes corresponding to the respective atoms constituting the repeat unit for composing the polymer to each other and an attach edge for attaching at least one of the nodes to the attaching node.

(2) Artificial neural network model unit 200

This is a configuration for estimating and calculating glass transition temperature property information about the polymer from the polymer graph data. The glass transition temperature property information about the polymer corresponds to $T_{g,inf}$ and K in Equation 1 described above.

Such an artificial neural network model unit is configured to receive polymer graph data and output an estimated value of the glass transition temperature property information about the corresponding polymer, and includes a polymer glass transition temperature property information estimation artificial neural network that is machine-trained by calculating an estimated value of glass transition temperature property information about a predetermined learning polymer from polymer graph data of the learning polymer, calculating an estimated Tg value from the calculated estimated value of the glass transition temperature property information about the learning polymer and the measured molecular weight information about the learning polymer, and updating its neural network parameters so that an error function defined as a difference between the estimated Tg value and the measured Tg value of the polymer in the training data is minimized.

(3) Glass transition temperature calculation unit 300

A glass transition temperature calculation unit is configured to calculate the glass transition temperature of the polymer according to [Equation 1] described above from the polymer glass transition temperature property information calculated by the artificial neural network model unit and the molecular weight data of the polymer input from the data input unit.

The graph data representation method for graphically representing the structure of a polymer according to the present invention, and the method and the system for finally calculating the accurate glass transition temperature of the polymer by estimating the glass transition temperature property information about the polymer from graph data of the polymer and reflecting the molecular weight information of the polymer in the estimated glass transition temperature property information about the polymer have been described above.

With the method and the system for calculating the glass transition temperature of the polymer, which reflects the molecular weight information, according to the present invention, by separately considering the effect of molecular weight, estimation adjustment according to the molecular weight may be possible, which has the effect of making it possible to build a model capable of accurate estimation.

For example, when actual Tg of each of samples of three types of polymers A, B, and C with similar structures and molecular weights of 2,000, 50,000, and 80,000, respectively, is similar, according to a model in the prior art, since only the structures of molecules are reflected without considering the molecular weights, learning and estimation are performed on samples with similar structures so that the estimated Tg values are similar regardless of molecular weights.

That is, when the actual Tg (glass transition temperature) of polymer A with molecular weight K is R_Tg_A(K) and the estimated Tg thereof is P_Tg_A(K), for R_Tg_A (2,000) ≈ R_Tg_B (50,000) ≈ R_Tg_C (80,000), the prior art trains a neural network and performs estimation to be P_Tg_A (2,000) ≈ P_Tg_B (50,000) ≈ P_Tg_C (80,000). In the case of the prior art, since the molecular weights are not reflected, even when the samples A, B, and C have the same molecular weight of 50,000, the prior art will estimate as P_Tg_A (50,000) ≈ P_Tg_B (50,000) ≈ P_Tg_C (50,000). However, when comparison is made with the same molecular weights for A, B, and C, in which R_Tg_A (2,000) ≈ R_Tg_B (50,000) ≈ R_Tg_C (80,000), according to Equation 1, in fact, R_Tg_A (50,000) > R_Tg_B (50,000) > R_Tg_C (50,000), and thus it is not possible to estimate the accurate glass transition temperature using the technology in the prior art.

In contrast, in the present invention, in the case of R_Tg_A (2,000) ≈ R_Tg_B (50,000) ≈ R_Tg_C (80,000), even

## EP 4 513 500 A1

when the neural network has be trained to be P_Tg_A (2,000) ≈ P_Tg_B (50,000) ≈ P_Tg_C (80,000), since the training of the neural network is performed by reflecting the molecular weights, neural network parameters for adjusting the effect of molecular structure are learned differently, and thus for the same molecular weight, the estimation is made as P_Tg_A (50,000) > P_Tg_B (50,000) > P_Tg_C (50,000).

In another case, when the molecular weights of polymers A, B, and C of similar structures are different and the actual Tgs are different, that is, when R_Tg_A (2,000) < R_Tg_B (50,000) < R_Tg_C (80,000), according to the prior art, since training is performed according to the molecular structures and the actual Tgs, which are label values, are given differently, it is learned and estimated that P_Tg_A (2,000) < P_Tg_B (50,000) < P_Tg_C (80,000) even when the polymers have similar structures. However, at this time, when the molecular weights of the polymers A, B, and C are the same, the actual Tg may be similar according to [Equation 1]. In this case, the estimated values should be derived as P_Tg_A (50,000) ≈ P_Tg_B (50,000) ≈ P_Tg_C (50,000), but since the model according to the prior art is trained based only on the molecular structures for the polymers A, B, and C with the same molecular weight, a problem occurs where it is estimated that P_Tg_A (50,000) < P_Tg_B (50,000) < P_Tg_C (50,000).

In contrast, according to the present invention, when R_Tg_A (2,000) < R_Tg_B (50,000) < R_Tg_C (80,000), since the training is performed by reflecting molecular weights, even when training is performed so that P_Tg_A (2,000) < P_Tg_B (50,000) < P_Tg_C (80,000), for the same molecular weight, it is not estimated that P_Tg_A (50,000) < P_Tg_B (50,000) < P_Tg_C (50,000), but is estimated that P_Tg_A (50,000) ≈ P_Tg_B (50,000) ≈ P_Tg_C (50,000).

As described above, according to the present invention, since the neural network model is trained to estimate the glass transition temperature by reflecting not only the molecular structure of the polymer but also the molecular weight information about the polymer, the effect of each of molecular structure information and molecular weight information may be learned, and thus the glass transition temperature may be more accurately estimated than the prior art.

Names of respective drawing symbols used in the present invention are as follows.

10     Node
20     Edge
30     Attach point
40     Attach edge
100    Data input unit
200    Artificial neural network model unit
300    Glass transition temperature calculation unit

### Claims

1. A method for generating a computer-implemented model that estimates a glass transition temperature of a selected polymer, the method comprising:

   a training data acquisition step of building a training data set with structure information about a plurality of learning polymers, known measured values of glass transition temperatures of corresponding polymers, and measured molecular weights of the corresponding polymers;

   a polymer glass transition temperature property information estimation artificial neural network construction step of constructing a polymer glass transition temperature property information estimation artificial neural network to receive structure information about a polymer and output an estimated value of the glass transition temperature property information about the polymer;

   a training data input step of inputting polymer structure information about the learning polymer and a measured value of a glass transition temperature of the learning polymer into the polymer glass transition temperature property information estimation artificial neural network; and

   a training step of the polymer glass transition temperature property information estimation artificial neural network, in which the neural network parameters of the polymer glass transition temperature property information estimation artificial neural network are updated based on a comparison result of an estimated value of the glass transition temperature of the learning polymer calculated by reflecting the measured molecular weights of the learning polymer in the training data set in the estimated value of the glass transition temperature property information about the learning polymer output by the polymer glass transition temperature property information estimation artificial neural network and a measured value of the glass transition temperature of the learning polymer.

2. The method of claim 1, wherein the glass transition temperature property information is a Tg,inf value and a K value in Equation 1 below;

[Equation 1]

$$T_g = T_{g,inf} - \frac{K}{M_n}$$

wherein Tg is a glass transition temperature of a polymer with a molecular weight Mn, $T_{g,inf}$ is a glass transition temperature when the molecular weight is infinite, K is a variable related to a free volume of the polymer, and Mn is a number average molecular weight of the polymer.

3. The method of claim 2, wherein the polymer structure information is converted into polymer graph data and input into the polymer glass transition temperature property information estimation artificial neural network, and
the polymer graph data comprises at least one of:

polymer graph node attachment data that is data representing an inter-attachment relationship between nodes corresponding to respective atoms constituting a repeat unit for composing the polymer and attaching nodes that are attached to an attach point that is a point where the repeat units are repeatedly attached among the nodes;
polymer node attribution information that is data representing attribute information about the nodes corresponding to the respective atoms constituting the repeat unit for composing the polymer and attributes of attaching nodes that are attached to the attach point that is a point where the repeat units are repeatedly attached among the nodes; and
polymer edge attribute information that is information representing attributes of an edge for attaching the nodes corresponding to the respective atoms constituting the repeat unit for composing the polymer to each other and an attach edge for attaching at least one of the nodes to the attaching node.

4. A computer-implemented method for calculating an estimated value of a glass transition temperature of a selected polymer, the computer-implemented method comprising:

a polymer glass transition temperature property information estimation artificial neural network acquisition step of generating a machine-trained graph artificial neural network to estimate glass transition temperature property information about the selected polymer based at least in part on a chemical structure related to the selected polymer;
a polymer graph data acquisition step of acquiring polymer graph data describing a chemical structure of the selected polymer as graph data;
a polymer graph input step of providing the acquired polymer graph data as input to the graph artificial neural network;
a polymer glass transition temperature property information acquisition step of receiving the polymer glass transition temperature property information of the selected polymer from output of the graph artificial neural network; and
a polymer glass transition temperature calculation step of calculating a glass transition temperature of the selected polymer by substituting molecular weight information about the selected polymer into the polymer glass transition temperature property information.

5. The computer-implemented method of claim 4, wherein the polymer graph data describing the chemical structure of the selected polymer as graph data comprises at least one of:

polymer graph node attachment data that is data representing an inter-attachment relationship between nodes corresponding to respective atoms constituting a repeat unit for composing the polymer and attaching nodes that are attached to an attach point that is a point where the repeat units are repeatedly attached among the nodes;
polymer node attribution information that is data representing attribute information about the nodes corresponding to the respective atoms constituting the repeat unit for composing the polymer and attributes of attaching nodes that are attached to the attach point that is a point where the repeat units are repeatedly attached among the nodes; and
polymer edge attribute information that is information representing attributes of an edge for attaching the nodes corresponding to the respective atoms constituting the repeat unit for composing the polymer to each other and an attach edge for attaching at least one of the nodes to the attaching node.

6. The computer-implemented method of claim 5, wherein the polymer graph input step of providing the acquired polymer graph data as input to the machine-trained graph artificial neural network comprises:

a polymer adjacency matrix construction step of constructing an adjacency matrix representing an inter-attachment relationship between two or more nodes representing respective atoms constituting the single molecule; and

a polymer adjacency matrix input step of inputting the constructed graph matrix into the graph artificial neural network, and

the polymer adjacency matrix further comprises attachment relationship information between the attaching node and the nodes.

7. The computer-implemented method of claim 4, wherein the graph artificial neural network is trained by:

calculating an estimated value of glass transition temperature property information about a learning polymer from structure information about a plurality of polymers; and

updating neural network parameters to minimize an error between an estimated value of a glass transition temperature calculated by reflecting a measured molecular weight of the learning polymer in the calculated estimated value of the glass transition temperature property information and a measured value of the glass transition temperature of the learning polymer.

8. The computer-implemented method of claim 4, wherein the polymer glass transition temperature property information estimation artificial neural network acquisition step comprises:

a step of acquiring, by one or more computing devices, training data including chemical structures of a plurality of exemplary polymers and measured values of glass transition temperatures and measured values of molecular weights of the exemplary polymers having the chemical structures of the exemplary polymers; and

a training step of acquiring graph data graphically describing the chemical structures of the exemplary polymers and inputting the graph data into the graph neural network, and training the graph neural network so that the estimated values of glass transition temperature property information about the exemplary polymers output by the graph neural network are output.

9. The computer-implemented method of claim 8, wherein the graph data graphically describing the chemical structures of the exemplary polymers comprises:

two or more pieces of node data representing respective atoms constituting a single molecule for composing the exemplary polymer;

one or more pieces of edge data representing coupling between the respective atoms;

attaching node data representing a point where the single molecules are repeatedly attached; and

attach edge data representing attachment between the atom and the attaching node.

10. The computer-implemented method of any one of claims 4 to 9, wherein the glass transition temperature property information is a Tg,inf value and K in Equation 2 below;

[Equation 2]

$$T_g = T_{g,inf} - \frac{K}{M_n}$$

wherein Tg is a glass transition temperature of a polymer with a molecular weight Mn, $T_{g,inf}$ is a glass transition temperature when the molecular weight is infinite, K is a variable related to a free volume of the polymer, and Mn is a number average molecular weight of the polymer.

11. The computer-implemented method of any one of claims 4 to 9, wherein

the polymer glass transition temperature calculation step comprises calculating the glass transition temperature of the polymer by substituting number average molecular weight information of the polymer into Equation 3 below;

[Equation 3]

$$T_g = T_{g,inf} - \frac{K}{M_n}$$

wherein Tg is a glass transition temperature of a polymer with a molecular weight Mn, $T_{g,inf}$ is a glass transition temperature when the molecular weight is infinite, K is a variable related to a free volume of the polymer, and Mn is a number average molecular weight of the polymer.

12. A glass transition temperature calculation system for polymers, comprising:

a data input unit that receives structure information about a selected polymer and molecular weight data of the polymer;
an artificial neural network model unit that calculates an estimated value of glass transition temperature property information about the polymer from the structure information about the polymer; and
a glass transition temperature calculation unit that calculates an estimated value of a glass transition temperature of the polymer from the estimated value of the glass transition temperature property information about the polymer and the molecular weight data of the polymer.

13. The glass transition temperature calculation system for polymers of claim 12, wherein the glass transition temperature property information about the polymer is a Tg,inf value and a K in Equation 4 below,

the molecular weight data of the polymer is Mn in Equation 4 below, and
the glass transition temperature calculation unit
calculates a glass transition temperature Tg from Equation 4 below,

[Equation 4]

$$T_g = T_{g,inf} - \frac{K}{M_n}$$

wherein Tg is a glass transition temperature of a polymer with a molecular weight Mn, $T_{g,inf}$ is a glass transition temperature when the molecular weight is infinite, K is a variable related to a free volume of the polymer, and Mn is a number average molecular weight of the polymer.

14. The glass transition temperature calculation system for polymers of claim 13, further comprising a data preprocessing unit that converts the structure information about the polymer into polymer graph data,

wherein the artificial neural network model unit is machine-trained to calculate the estimated value of the glass transition temperature property information about the polymer from the polymer graph data, and
the polymer graph data comprises at least one of:

polymer graph node attachment data that is data representing an inter-attachment relationship between nodes corresponding to respective atoms constituting a repeat unit for composing the polymer and attaching nodes that are attached to an attach point that is a point where the repeat units are repeatedly attached among the nodes;
polymer node attribution information that is data representing attribute information about the nodes corresponding to the respective atoms constituting the repeat unit for composing the polymer and attributes of attaching nodes that are attached to the attach point that is a point where the repeat units are repeatedly attached among the nodes; and
polymer edge attribute information that is information representing attributes of an edge for attaching the nodes corresponding to the respective atoms constituting the repeat unit for composing the polymer to each other and an attach edge for attaching at least one of the nodes to the attaching node.

15. The glass transition temperature calculation system for polymers of claim 12, wherein the artificial neural network model unit includes a polymer glass transition temperature property information estimation artificial neural network configured to receive structure information about a polymer and output an estimated value of the glass transition temperature property information about the polymer,

the polymer glass transition temperature property information estimation artificial neural network is machine-trained by, using structure information about a predetermined learning polymer and a measured value of a glass transition temperature and a measured value of a molecular weight of the learning polymer as training data, calculating an estimated value of the glass transition temperature property information about the learning polymer from the structure information about the learning polymer, calculating the estimated value of the glass transition temperature of the learning polymer using the estimated value of the glass transition temperature property information and the measured value of the molecular weight and updating neural network parameters of the polymer glass transition temperature property information estimation artificial neural network by comparing the estimated value with the measured value of the glass transition temperature of the learning polymer.

16. The glass transition temperature calculation system for polymers of claim 15, wherein the training data contains data sets including polymer graph data containing structure information about a sample polymer, a measured Tg value of the sample polymer, and a molecular weight (Mn) of the sample polymer, and

at least a predetermined number of the data sets are data sets of polymers with similar structures and different molecular weights and data sets of polymers with different structures and the same molecular weight.

**Fig. 1**

monomer A                          polymer A

(a)

monomer B                          polymer B

(b)

Fig. 2

Polymer graph representation

(a)

(b)

Fig. 3

(a)

(b)

**Fig. 4**

POLYMER CHEMICAL STRUCTURE INFORMATION /POLYMER MEASURED Tg /POLYMER MOLECULAR WEIGHT

T10 : PREPARE TRAINING DATA

POLYMER GRAPH DATA /POLYMER MEASURED Tg /POLYMER MOLECULAR WEIGHT

T20 : INPUT TO ARTIFICIAL NEURAL NETWORK MODEL

ARTIFICIAL NEURAL NETWORK

T30 : UPDATE NEURAL NETWORK MODEL PARAMETER

POLYMER GLASS TRANSITION TEMPERATURE PROPERTY INFORMATION OF TRAINING DATA

(a)

POLYMER CHEMICAL STRUCTURE INFORMATION

S10 : POLYMER GRAPH DATAFICATION PREPROCESSING PROCESS

POLYMER GRAPH DATA

S20 : INPUT TO ARTIFICIAL NEURAL NETWORK MODEL

ARTIFICIAL NEURAL NETWORK

S30 : ESTIMATE POLYMER GLASS TRANSITION TEMPERATURE PROPERTY INFORMATION

POLYMER GLASS TRANSITION TEMPERATURE PROPERTY INFORMATION

S40 : REFLECT MOLECULAR WEIGHT INFORMATION

POLYMER GLASS TRANSITION TEMPERATURE

(b)

**Fig. 5**

POLYMER STRUCTURE INFORMATION,
POLYMER MOLECULAR WEIGHT INFORMATION

```
┌──────────────────────────┐
│    DATA INPUT UNIT        │
│        (100)              │
└──────────────────────────┘
```

POLYMER GRAPH DATA

```
┌──────────────────────────┐
│ ARTIFICIAL NEURAL NETWORK │
│      MODEL UNIT           │
│        (200)              │
└──────────────────────────┘
```

POLYMER GLASS
TRANSITION TEMPERATURE
PROPERTY INFORMATION

POLYMER MOLECULAR
WEIGHT INFORMATION

```
┌──────────────────────────┐
│ GLASS TRANSITION TEMPERATURE │
│    CALCULATION UNIT       │
│        (300)              │
└──────────────────────────┘
```

POLYMER GLASS TRANSITION
TEMPERATURE

| | INTERNATIONAL SEARCH REPORT | International application No. |
|---|---|---|
| | | **PCT/KR2023/021741** |

**A. CLASSIFICATION OF SUBJECT MATTER**

**G16C 20/70**(2019.01)i; **G16C 20/40**(2019.01)i; **G16C 20/80**(2019.01)i; **G06N 3/04**(2006.01)i; **G06N 3/08**(2006.01)i

According to International Patent Classification (IPC) or to both national classification and IPC

**B. FIELDS SEARCHED**

Minimum documentation searched (classification system followed by classification symbols)

G16C 20/70(2019.01); B60C 19/00(2006.01); C08B 11/02(2006.01); C08B 3/00(2006.01); G06N 20/00(2019.01); G16C 10/00(2019.01); G16Z 99/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고분자(polymer), 유리전이온도(glass transition temperature), 구조정보(structure information), 분자량(molecular weight), 인공 신경망(artificial neural network)

**C. DOCUMENTS CONSIDERED TO BE RELEVANT**

| Category* | Citation of document, with indication, where appropriate, of the relevant passages | Relevant to claim No. |
|---|---|---|
| A | VOLGIN, Igor V. et al. Machine Learning with Enormous "Synthetic" Data Sets: Predicting Glass Transition Temperature of Polyimides Using Graph Convolutional Neural Networks. ACS OMEGA. 17 November 2022, Vol. 7, Issue 48, pp. 43678–43691.<br>See pages 43680-43687. | 1-16 |
| A | JP 2022-034121 A (DAICEL CORP.) 03 March 2022 (2022-03-03)<br>See paragraphs [0015]-[0095]. | 1-16 |
| A | JP 2022-088015 A (SUMITOMO RUBBER IND LTD.) 14 June 2022 (2022-06-14)<br>See paragraphs [0006]-[0027]. | 1-16 |
| A | JP 2022-013310 A (YOKOHAMA RUBBER CO., LTD.) 18 January 2022 (2022-01-18)<br>See paragraphs [0018]-[0040]. | 1-16 |
| A | KR 10-2214634 B1 (KOREA RESEARCH INSTITUTE OF CHEMICAL TECHNOLOGY) 10 February 2021 (2021-02-10)<br>See paragraphs [0051]-[0090]. | 1-16 |

☐ Further documents are listed in the continuation of Box C.    ☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **23 April 2024** | **24 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT
Information on patent family members

International application No.

**PCT/KR2023/021741**

| Patent document cited in search report | | | Publication date (day/month/year) | Patent family member(s) | | | Publication date (day/month/year) |
|---|---|---|---|---|---|---|---|
| JP | 2022-034121 | A | 03 March 2022 | None | | | |
| JP | 2022-088015 | A | 14 June 2022 | None | | | |
| JP | 2022-013310 | A | 18 January 2022 | None | | | |
| KR | 10-2214634 | B1 | 10 February 2021 | KR 10-2020-0052393 | A | | 15 May 2020 |

Form PCT/ISA/210 (patent family annex) (July 2022)

**REFERENCES CITED IN THE DESCRIPTION**

**Patent documents cited in the description**

- KR 20210042777 A

- KR 20210110539 A

**Non-patent literature cited in the description**

- Machine-learning predictions of polymer properties with Polymer Genome. *Journal of Applied Physics*, 2020, vol. 128, 171104

- Machine learning discovery of high-temperature polymers. *Matter*, 05 May 2021, vol. 4 (5), 1454-1456